(19) Europäisches Patentamt
European Patent Office
Office européen des brevets

(11) **EP 4 167 922 B1**

(12) **EUROPEAN PATENT SPECIFICATION**

(45) Date of publication and mention
of the grant of the patent:
**05.03.2025 Bulletin 2025/10**

(21) Application number: **21737129.3**

(22) Date of filing: **18.06.2021**

(51) International Patent Classification (IPC):
*A61K 8/19* (2006.01)    *A61K 8/73* (2006.01)
*A61K 8/02* (2006.01)    *A61K 8/22* (2006.01)
*A61K 8/24* (2006.01)    *A61K 8/38* (2006.01)
*A61K 8/81* (2006.01)    *A61Q 11/00* (2006.01)

(52) Cooperative Patent Classification (CPC):
**A61Q 11/00; A61K 8/0208; A61K 8/0241;
A61K 8/19; A61K 8/22; A61K 8/24; A61K 8/38;
A61K 8/73; A61K 8/733; A61K 8/8176;**
A61K 2800/413; A61K 2800/88

(86) International application number:
**PCT/GB2021/051554**

(87) International publication number:
**WO 2021/255474 (23.12.2021 Gazette 2021/51)**

(54) **TOOTH WHITENING AND TOOTH SENSITIVITY STRIP OR FILM**

ZAHNBLEICHUNGS- UND ZAHNEMPFINDLICHKEITSSTREIFEN ODER -FOLIE

BANDE OU FILM POUR BLANCHIMENT DES DENTS ET SENSIBILITÉ DES DENTS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.06.2020 GB 202009404**

(43) Date of publication of application:
**26.04.2023 Bulletin 2023/17**

(73) Proprietor: **Bsolve Limited**
**Glasgow G72 0FB (GB)**

(72) Inventors:
• **CULLEN, John Edward**
**Glasgow G72 0FB (GB)**

• **LIVINGSTONE, Mark Alexander**
**Glasgow G72 0FB (GB)**
• **CRICHTON, Robert**
**Glasgow G72 0FB (GB)**
• **MACFARLANE, Melanie**
**Glasgow G72 0FB (GB)**

(74) Representative: **HGF**
**HGF Limited**
**1 City Walk**
**Leeds LS11 9DX (GB)**

(56) References cited:
**WO-A1-2006/009737    WO-A2-2005/016298
GB-A- 2 562 800    US-A1- 2008 171 000
US-A1- 2018 289 606**

**Description**

**FIELD OF THE INVENTION**

The present invention relates to an orally dissolvable strip or film for tooth whitening and/or tooth sensitivity. In particular, the present invention relates to a thin orally dissolvable or substantially dissolvable strip or film for tooth whitening and/or tooth sensitivity comprising hydroxyapatite that may be in a nano and/or non-nano form.

**BACKGROUND**

Human teeth naturally exhibit a variety of colours, which can be influenced by a number of factors, such as diet and medication. For instance, a number of foods, such as berries, and drinks, such as tea and red wine, contain chromogens which can stain teeth. Tobacco may also darken teeth. Other substances, such as acidic fruits and drinks, can promote staining by eroding dental enamel, softening teeth and making it easier for chromogens to attach. Consequently, there is a desire to whiten teeth to remove such staining.

General dentin hypersensitivity (DH) is uncomfortable for the sufferer. There are toothpastes on the market that are used for treatment and prevention of dentin hypersensitivity (DH). However, these products have deficiencies in relation to contact time with sensitive teeth. They are also applied to a general area as opposed to the specific areas of concern in the mouth and may require prolonged use before the user notices the benefits.

The use of toothpaste generally requires the use of a sink and toothbrush, which can sometimes be inconvenient. In addition, a whitening toothpaste will come into contact with oral mucosa and may cause oral sensitivity or pain.

The creation of a strip which contains an active ingredient that can be used to treat dentin hypersensitivity (DH) may be used to solve the problems associated with residence time (tailor the dissolution time) and have a targeted application area. For example, a user can place a strip on an affected area and reduce contact with oral mucosa. In addition, other ingredients that freshen breath and improve overall oral health can also be incorporated. Some teeth whitening and/or teeth sensitivity strips are disclosed in WO 2006/009737, WO 2005/016298, US 2008/171000, US 2018/289606, and GB 2562800.

It is desirable to have white teeth. Certain whitening agents such as $H_2O_2$ and carbamide peroxide are known to increase incidence or severity of dentin hypersensitivity (DH). Treatment with toothpaste is inconvenient and requires the user to use two separate products.

The present applicant has developed a thin film strip that contains $H_2O_2$ and an agent that reduces severity of dentin hypersensitivity (DH). A consumer has the benefit of whiter teeth but with reduced level of sensitivity.

It is an object of at least one aspect of the present invention to obviate or at least mitigate one or more of the aforementioned problems.

It is a further object of the present invention to provide a dissolvable or substantially dissolvable tooth whitening strip or film which reduces and/or minimises severity of dentin hypersensitivity (DH).

It is a yet further object of the present invention to provide a dissolvable or substantially dissolvable tooth whitening strip or film which may be used to whiten teeth with a reduced level of sensitivity.

There is also described an improved method of whitening teeth (that is not in accordance with the claims), which reduces and/or minimises severity of dentin hypersensitivity (DH).

There is also described an improved cosmetic method of whitening teeth (that is not in accordance with the claims), which reduces and/or minimises severity of dentin hypersensitivity (DH).

**SUMMARY OF INVENTION**

According to a first aspect of the present invention there is provided a tooth whitening and/or tooth sensitivity strip or film comprising:

1 - 20 wt.% hydroxyapatite (HAP);
at least one or a combination of polyvinylpyrrolidone (PVP) polymers wherein the amount of the PVP polymers is greater than 35 wt.% of the strip or film;
at least one or a combination of polycarbophil(s), 1-5 wt. % of the strip or film; and
phthalimido peroxy caproic acid (PAP) with the concentration of: 0.01 wt.% to 5 wt.% of the strip or film.

The present invention therefore provides a tooth whitening and/or tooth sensitivity strip or film which overcomes previous problems.

The wt.% in the present application may be defined as dry weight of the tooth whitening and/or tooth sensitivity strip or film.

A preferred feature of the strip or film is that the strip or film prevents degradation of the hydroxyapatite (HAP) during use and when inserted into a person's mouth.

The hydroxyapatite (HAP) may be in a nano and/or non-nano form.

The hydroxyapatite (HAP) may be in a nano crystalline form.

The hydroxyapatite (HAP) may be in the form of a paste such as nanoXIM (Trade Mark) paste.

The hydroxyapatite (HAP) may be in the form of Pentacalcium hydroxide triphosphate e.g. $Ca_{10}(PO_4)_6 (OH)_2$.

In particular, the present invention provides a thin dissolvable strip or film which can reduce/prevent dental hypersensitivity (DH). DH is a common side effect of peroxide teeth whitening. However, by incorporating a desensitising ingredient into a thin film alongside a whitening agent it is possible to prevent this undesirable effect.

The present invention relates to the incorporation of a range of desensitising ingredients into thin strips or films selected from any one of or combination of the following: nano-hydroxyapatite paste; hydroxyapatite (HAP) powder; potassium oxalate and/or Vitryxx™ bioactive glass.

This document details an internal study of the whitening efficacy of strips containing (nominally) about 6 - 10 wt.% hydrogen peroxide (e.g. about 6% hydrogen peroxide) and either about 6 - 10 wt.% nano-hydroxyapatite paste (e.g. about 6.5 wt.% nano-hydroxyapatite paste) or about 6 - 10 wt.% potassium oxalate (e.g. about 6.5 wt.% potassium oxalate). The applicant's standard 6% hydrogen peroxide formulation was used to provide a positive control. In addition, there are external whitening study data that outlines the efficacy of a HAP powder containing hydrogen peroxide strip against a non-HAP containing competitor.

The hydroxyapatite (HAP) may be in the form of nano-hydroxyapatite (n-HAP) that has a high loading of n-HAP.

There is also described loading of n-HAP (that is not in accordance with the claims), that may be any of the following: greater than about 10 wt.%; or greater than about 15 weight percent; or greater than about 20 wt.%.

Furthermore, the loading of n-HAP may be any of the following: about 5 - 20 wt.%; about 10 - 20 wt.%; or about 15 - 20 wt.%.

The hydroxyapatite (HAP) (e.g. n-HAP) may be in the form of a powder formulation. The powder formulation may comprise HAP in the following amounts: about 1 - 20 wt.%; about 1 - 10 wt.%; about 5 - 15 wt.%; about 5 - 20 wt.%; about 1 - 15 wt.%.

There is also described strip formulations (that are not in accordance with the claims), that may comprise whitening agents such as e.g. phthalimidoperoxycaproic acid (PAP) and or hydrogen peroxide and optionally in combination with one or more polyphosphates such as sodium tripolyphosphate or sodium hexametaphosphate.

The tooth whitening and/or tooth sensitivity strip or film of the present invention may be placed directly onto a tooth area effected with dentin hypersensitivity (DH).

The tooth whitening and/or tooth sensitivity strip or film of the present invention also provides the advantage of requiring no need for equipment and offers added convenience. The formulation can also be altered to include whitening and other oral health actives.

In addition, the tooth whitening and/or tooth sensitivity strip or film of the present invention may be used to provide a dose and dissolution time which can be altered to suit needs.

A potential embodiment of the present invention may deliver a "high" dose of anti-sensitivity active in a rapid dissolving composition.

Alternatively, another embodiment may include a "low" dose delivered over an extended period.

The dissolvable strip or film may comprise hydrogen peroxide (i.e. $H_2O_2$) the range of about 6 - 20 wt.% or preferably about 6 - 10 wt.%. Alternatively, other whitening agents such as urea peroxide may be used.

In addition, the dissolvable strip or film may comprise any one of a combination of the following: calcium sodium phosphosilicate such as Vitryxx™; potassium oxalate; the amino acid arginine and related compounds such as arginine bicarbonate; potassium nitrate; and various blends with optional inclusion of a source of fluoride or alternative sources of calcium, sodium, potassium and / or phosphate ions such as calcium phosphate, dicalcium phosphate, tricalcium phosphate, calcium carbonate, calcium silicate, calcium citrate, calcium monofluorophosphate, calcium sulphate, pentasodium triphosphate, sodium hexametaphosphate, sodium metaphosphate, sodium monofluorophosphate, sodium phosphate, sodium polyphosphate, sodium trimetaphosphate, disodium pyrophosphate, tetrasodium pyrophosphate, trisodium phosphate, potassium phosphate, dipotassium phosphate, pentapotassium triphosphate, potassium carbonate, potassium citrate, potassium fluoride, potassium silicate, potassium fluorosilicate, potassium metaphosphate, potassium monofluorophosphate, tetrapotassium pyrophosphate, tripotassium phosphate.

There is also described a dissolvable or substantially dissolvable teeth whitening strip or film (that is not in accordance with the claims), comprising hydrogen peroxide ($H_2O_2$) with the concentration of hydrogen peroxide ($H_2O_2$) of: about 10 wt.% to 30 wt.% of the strip or film; about 15 wt.% to 30 wt.% of the strip or film; about 10 wt.% to 20 wt.% of the strip or film; or about 10 wt.% to 15 wt.% of the strip or film.

The teeth whitening strips or film may comprise a range of other components including any one of or combination of the following:

- one or more polyphosphates,
- one or more water soluble film-forming polymers;
- one or more plasticizers; and/or
- one or more emulsifiers.

The film or strip of the present invention has a thickness ranging from: 50 $\mu$m to 500 $\mu$m.

There is also described a film or strip that may have a thickness ranging from: about 50 $\mu$m to about 250 $\mu$m; or about 50 $\mu$m to about 100 $\mu$m; or about 100 $\mu$m to about 200 $\mu$m.

There is also described a film or strip (that is not in accordance with the claims), wherein the soluble film-forming polymer may be one or more of the group comprising: polyvinyl pyrrolidone; pullulan; pectin; starch; dextrin; chitosan; alginic acid; salts of alginic acid, polyalkylene oxides and cellulose derivatives.

Suitable cellulose derivatives include carboxyalkyl cellulose or a salt thereof and hydroxyalkyl cellulose or a salt thereof, in which the alkyl group of the carboxyalkyl cellulose or the hydroxyalkyl cellulose is independently selected from $C_{1-5}$ alkyl, preferably methyl, ethyl or propyl. A preferred hydroxyalkyl cellulose is hydroxypropyl cellulose.

There is also described a film or strip (that is not in accordance with the claims), wherein preferably, the water soluble film-forming polymer may be selected from the group comprising polyvinyl pyrrolidone, pullulan, pectin, starch, carboxyalkyl cellulose or a salt thereof, hydroxyalkyl cellulose or a salt thereof, in which the alkyl group is independently selected from $C_{1-5}$ alkyl, alginic acid, salt of alginic acid, polyalkylene glycol in which the alkylene group has 2 or 3 carbon atoms and copolymers thereof, polyacrylic acid or a salt thereof and combinations thereof.

The water soluble film-forming polymer may be the polymer in the hydrogen peroxide-polymer complex which can form the dental bleaching agent. For instance, polyvinyl pyrrolidone may be present in a complex with hydrogen peroxide as the dental bleaching agent.

The one or more film forming polymers may be present in the film or strip (that is not in accordance with the claims), in a total amount of from: about 40% to about 95% by weight; about 50% to about 95% by weight; about 60% to about 95% by weight; 50% to about 80% by weight; about 50% to about 70% by weight.

Preferably, the one or more film forming polymers may be present in a total amount of from about 50% to about 90% by weight. More preferably, the one or more film forming polymers may be present in a total amount of from about 60% to about 85% by weight. Still more preferably, the one or more film forming polymers may be present in a total amount of from about 65% to about 80% by weight.

In one embodiment, the one or more plasticizers are selected from the group comprising a polyol such as glycerol, polyalkylene glycol, polyalkylene glycol monomethyl ether, monosaccharide, oligosaccharide, sorbital and sorbitan, in which the alkylene groups are independently selected from from $C_{1-5}$ alkylene, preferably methylene, ethylene or propylene. Preferred polyalkylene glycols are one or both of polyethylene glycol and polypropylene glycol. A preferred polyalkylene glycol monomethyl ether is polyethylene glycol monomethyl ether. A preferred plasticizer is glycerol.

The one or plasticizers may be present in the film or strip in a total amount of from about 0.1% to about 15% by weight. Preferably the one or plasticizers may be present in a total amount of from about 1% to about 12% by weight. More preferably, the one or plasticizers may be present in the film in a total amount of from about 3% to about 10% by weight.

The tooth whitening / dental hypersensitivity film may comprise one or more emulsifiers. In one embodiment, the one or more emulsifiers may be selected from ionic emulsifiers and non-ionic emulsifiers. In another embodiment the one or more emulsifiers may be selected from the group comprising a fatty acid derivative, a lecithin and a polysorbate.

Preferably the emulsifier, such as the non-ionic emulsifier, may be selected from the group comprising a saturated fatty acid derivative, a lecithin or a polysorbate. The fatty acids may be saturated or unsaturated. More preferably the non-ionic emulsifier comprises one or both of at least one unsaturated fatty acid such as oleic acid and/or linoleic acid and optionally at least one saturated fatty acid such as palmitic acid and/or stearic acid or a polysorbate.

More preferred emulsifiers, such as non-ionic emulsifiers, are selected from fatty acids, which may be saturated or unsaturated and a polysorbate. More preferably the emulsifier, such as the non-ionic emulsifier, comprises one or both of (i) at least one unsaturated fatty acid such as oleic acid and/or linoleic acid and optionally at least one saturated fatty acid such as palmitic acid and/or stearic acid and (ii) a polysorbate. A polysorbate is a polyethoxylated ester of sorbital, sorbitan and isosorbide.

Preferred saturated fatty acid derivatives include sucrose esters of saturated fatty acids; mono-, di- or tri-glycerides of saturated fatty acids; or sorbitan esters of saturated fatty acids.

The one or more emulsifiers may be present in the film in a total amount of from about 0.1% to 10% by weight. Preferably the one or more emulsifiers are present in a total amount of from about 1% to 5% by weight, more preferably from about 2% to 4% by weight.

In one embodiment, the tooth whitening film may further comprise water. The water may be present in the film in an amount of less than or equal to about 15% by weight, particularly from about 0.1% to 15% by weight. Typically, the tooth whitening film may further comprise water in an amount of from about 3% to 12% by weight, preferably from about 5% to 10% by weight.

In one embodiment, the tooth whitening film may further comprise one or more optional components selected from the group comprising colourant, gelling agent, flavouring, sweetener, acidifier, antioxidant, dissolution extender and chelating agent.

The gelling agent may be a hydrocolloid adhesive agent.

Other components of the tooth whitening film, such as some types of water soluble film forming polymer like polyvinyl pyrrolidone and certain polysaccharides like hydroxypropyl cellulose may exhibit gelling. For the purpose of the present disclosure, when a gelling agent is present as an optional further component of the tooth whitening film, this is considered separately from, and in addition to any of the other components which may exhibit gelling behaviour.

The flavouring may be selected from the group comprising menthol, peppermint and an alkyl alkanoates, in which the alkyl group may be straight chained or branched and may comprise from 1 to 8 carbon atoms, and the alkanoate group may comprise from 1 to 5 carbon atoms.

The sweetener may be selected from one or more of the group comprising aspartame, acesulfame K, sucralose, cyclamate, erythritol, mannitol, sorbital, stevia and xylitol.

The acidifier may be phosphoric acid.

The antioxidant may be one or more selected from the group comprising tocopherol (vitamin E), tertiary-butylhydroquinone (TBHQ), butylated hydroxyanisole (BHA) butylated hydroxytoluene and ethylenediaminetetraacetic acid or a salt thereof.

The introduction of polycarbophils (e.g. Noveon, Registered Trade Mark) at low amounts (e.g. about 1- 5 wt.% or about 2 - 3 wt.%) allows for slowing of the dissolution rate of the composition and so increases whitening efficacy.

The Noveon (Registered Trade Mark) may be Noveon AA-1 polycarbophil USP with a Brookfield viscosity of 2,000 -12,000 (measured at Brookfield RVT, 20 rpm, neutralised to pH 7.3 - 7.8).

The polycarbophil may comprise the metal salt of polyacrylic acid cross-linked with a glycol.

In particular embodiments, the polycarbophil may comprise the calcium salt of polyacrylic acid cross-linked with divinyl glycol having a viscosity of, for example, between about 2,000 - 12,000 mPa.s for about a 0.2% solution.

The polycarbophil may be in the form of the metal salt such as polycarbophil calcium. The polycarbophil may be formed as a synthetic polymer of polyacrylic acid cross-linked with divinyl glycol, with, for example, calcium as a counter-ion.

The chelating agent may be ethylenediaminetetraacetic acid or a salt thereof. Chelating agents can be used to sequester heavy metal ions, thereby preventing the degradation of peroxy carboxylic acids.

Other ingredients may be selected for anti-microbial or anti-bacterial properties that afford general improved oral health such as cetylpyridinium chloride or zinc acetate.

In another embodiment, the tooth whitening / dental hypersensitvity film may be a single layer.

In a further embodiment, the tooth whitening film / dental hypersensitvity is free of any other layer or layers such as barrier layers or supporting substrates.

In another embodiment, the film, which may be used without a dental aligner, has a thickness in the range of from about 50 to 250 micrometers, more preferably the film has a thickness in the range of from about 100 to 200 micrometers, still more preferably about 150 micrometers.

Preferably, the film, which may be used without a dental aligner, has a length of from about 50 to 70 mm, more preferably about 60 mm. Preferably the film has a width of from about 10 to 20 mm, more preferably about 15 mm.

The film, which may be used without a dental aligner, may have a weight in the range of from about 60 to 250 mg, preferably from about 100 to 200 mg, more preferably about 150 mg.

In another embodiment, the film, which may be used with a dental aligner, has a thickness in the range of from about 50 to 150 micrometers, more preferably the film has a thickness in the range of from about 60 to 130 micrometers, still more preferably about 70 - 125 micrometers.

In a second aspect, there is provided a process for the manufacture of a tooth whitening / dental hypersensitvity strip or film, the process comprising at least the steps of:

- mixing at least one or a combination of polyvinylpyrrolidone (PVP) polymers wherein the amount of the polyvinylpyrrolidone (PVP) polymers is greater than about 35 wt.% of the film or strip; and
- 1- 20 wt.% hydroxyapatite (HAP);
- at least one or a combination of polycarbophils, 1-5 wt. % of the strip or film; and
- phthalimido peroxy caproic acid (PAP) with the concentration of: 0.01 wt.% to 5 wt.% of the strip or film;
- with water to provide an aqueous tooth whitening liquid;
- applying the aqueous tooth whitening liquid to a substrate to provide a substrate carrying the aqueous tooth whitening liquid;
- drying the aqueous tooth whitening liquid to provide a tooth whitening film on the substrate, said film having a thickness from 50 $\mu$m to 500 $\mu$m.

There is also described a process (that is not in accordance with the claims), that may comprise a source of a teeth whitening agent such as hydrogen peroxide.

In one embodiment, the aqueous tooth whitening / dental hypersensitivity liquid comprises greater than about 60 wt.% water (on the basis of the total weight of the tooth whitening / dental hypersensitivity liquid, which includes the water present), preferably from about 70 wt.% to 85 wt.% water. Thus, the aqueous tooth whitening / dental hypersensitivity liquid may comprise about 40 wt.% or less of the components forming the tooth whitening film / dental hypersensitivity, preferably from about 15 wt.% to 30 wt.%.

In one embodiment, the drying step comprises:

- drying the aqueous tooth whitening / dental hypersensitivity liquid in air at a temperature of less than about 60°C.

In a further embodiment, the drying of the aqueous tooth whitening liquid / dental hypersensitivity in air is carried out at a temperature in the range of from about 40 °C to less than 60°C, preferably from about 40 °C to 55 °C.

In a further embodiment, the drying may be carried out at a rate in the range of from about 0.2 to 2.0 m/min, preferably in a range of from about 0.5 to 1.5 m/min, for instance by passing the aqueous tooth whitening / dental hypersensitivity liquid on the substrate through a dryer, such as a drying oven, hot air stream etc.

In a further embodiment, the drying step is carried out for a period of about 80 minutes or less, preferably for a period of about 35 minutes or less, more preferably for a period of about 25 minutes or less. The drying step may be carried out for a period of at least about 8 minutes, preferably for a period of at least about 15 minutes, more preferably for a period of at least about 20 minutes. Thus, the drying step may be carried out for a period of from about 8 to 80 minutes, preferably from about 15 to 35 minutes and more preferably from 20 to 25 minutes.

In another embodiment, the mixing step may be carried out under high shear, for instance at a shear rate of greater than about 500 s$^{-1}$, preferably greater than about 1000 s$^{-1}$, up to a maximum of about 8000 s$^{-1}$. Preferably the shear rate is in a range of from about 1000 s$^{-1}$ to 4000 s$^{-1}$.

In one embodiment, the mixing step further comprises mixing one or more further components selected from the group comprising colourant, gelling agent, flavouring, sweetener, acidifier, antioxidant and chelating agent.

In a third aspect, there is provided a tooth whitening / dental hypersensitivity strip or film obtainable by the process of the second aspect and its embodiments.

Herein is also described a method of bleaching teeth (that is not in accordance with the claims), said method comprising at least the step of:

- applying a tooth whitening strip film according to the first, second or third aspects to one or more teeth of a subject.

The method (that is not in accordance with the claims), may be a cosmetic method, particularly a solely cosmetic method.

The film or strip may be applied to the front surface of one or more teeth and/or over the front surface of one or more teeth, chewing and back surfaces of one or more teeth.

## BRIEF DESCRIPTION OF DRAWINGS

Embodiments of the present invention will now be described, by way of example only, with reference to the accompanying drawings in which:

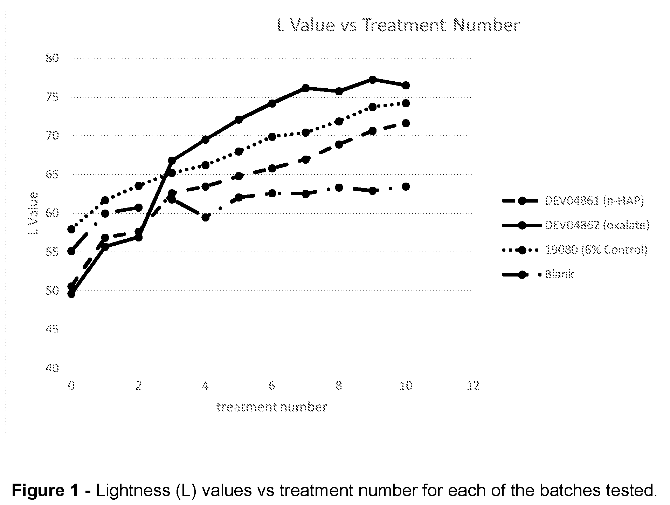

Figure 1 is a representation of lightness (L) values vs treatment number for a range of batches tested;
Figure 2 is a table showing a range of compositions;
Figures 3A and 3B show Tables 3A and 3B showing a range of compositions; and
Figure 4 shows Table 4 which is a range of further data for the compositions shown in Tables 3A and 3B.

## DETAILED DESCRIPTION

The present invention relates to a tooth whitening strip or film. The present invention also relates to a thin orally dissolvable or substantially dissolvable strip or film for tooth whitening and/or tooth sensitivity comprising hydroxyapatite (e.g. nanoXIM). The hydroxyapatite may be in a nano and/or non-nano form.

In particular, the present invention relates to a dissolvable or substantially dissolvable tooth whitening strip or film being phthalimidoperoxycaproic acid (PAP) containing strips or films.

The present invention also relates to a process for the manufacture of a dissolvable or substantially dissolvable tooth whitening / dental hypersensitivity strip or film. Herein is also described a method of using said strip or film (that is not in accordance with the claims), to whiten and/or bleach teeth. The method may be a cosmetic method to whiten and/or bleach teeth.

Plasticizer

In the present invention a plasticizer may be present in the tooth whitening film. Preferably, the plasticizer should be a physiologically acceptable plasticizer.

The one or more plasticizers may be selected from the group comprising a polyol such as glycerol, polyalkylene glycol in which the alkylene groups may be independently selected from from $C_{2-5}$ alkylene, polyalkylene glycol monomethyl ether in which the alkylene groups are independently selected from from $C_{2-5}$ alkylene, monosaccharide, oligosaccharide, sorbital and sorbitan, preferably ethylene or propylene. Preferred polyalkylene glycols are one or both of polyethylene glycol and polypropylene glycol. A preferred polyalkylene glycol monomethyl ether is polyethylene glycol monomethyl ether.

The plasticizer may be a hydrophilic plasticizer, such as one or more of the group comprising a polyol such as glycerol, polyethylene glycol, polyethylene glycol monomethyl ether, propylene glycol, sorbital and sorbitan. The preferred plasticizer is glycerol.

The one or plasticizers may be present in the film in a total amount of from about 0.1% to about 15% by weight. Preferably the one or plasticizers may be present in a total amount of from about 1% to about 12% by weight. More preferably, the one or plasticizers may be present in the film in a total amount of from about 3% to about 10% by weight or in a range of from about 1% to about 5% by weight.

Emulsifier

The tooth whitening film may comprise one or more emulsifiers. The one or more emulsifiers may be selected from ionic emulsifiers and/or non-ionic emulsifiers.

Preferably the non-ionic emulsifier is selected from the group comprising a saturated fatty acid derivative, a lecithin or a polysorbate. The fatty acids may be saturated or unsaturated. More preferably the non-ionic emulsifier comprises one or both of at least one unsaturated fatty acid such as oleic acid and/or linoleic acid and optionally at least one saturated fatty acid such as palmitic acid and/or stearic acid or a polysorbate.

Polysorbates are polyethoxylated esters of sorbital, sorbitans and isosorbide. Preferred saturated fatty acid derivatives include sucrose esters of saturated fatty acids; mono-, di- or tri-glycerides of saturated fatty acids; or sorbitan esters of saturated fatty acids. A preferred emulsifier is Sorbital, such as T80 supplied by Azelis.

The one or more emulsifiers may be present in the film in a total amount of from about 0.1% to 10% by dry weight of the tooth whitening film, preferably in the range of from about 2.0 to 2.5 % by weight.

Optional components

The tooth whitening film may further comprise one or more optional components selected from the group comprising colourant, gelling agent, flavouring, sweetener, acidifier, antioxidant, anti-microbial/bacterial and chelating agent.

The gelling agent may be a hydrocolloid. Typically, the gelling agent may be a hydrocolloid which also functions as an adhesive agent. As used herein, the term "hydrocolloid" means a hydrophilic polymer, which may be of any origin such as plant, animal, microbial or synthetic, which contains hydrophobic groups and forms a gel in the presence of water, that is, a heterogeneous system with a solid hydrocolloid network containing a liquid water phase. The hydrocolloid adhesive agent may be selected from a natural gum, a polyacrylic acid or a poly alkylacrylic acid in which the alkyl group is methyl or ethyl. The alkyl group may be methyl or ethyl i.e. poly methacrylic acid or poly ethacrylic acid. The natural gum may be selected from alginic acid and its salts, agar, carrageenan, tragacanth and polysaccharide gums. More preferably, when the hydrocolloid gelling agent is a natural gum, it may be tragacanth gum comprising a mixture of water-soluble and water-insoluble polymers, particularly polysaccharides, such as a mixture of tragacanthin and bassorin polymers. The bassorin polymer may be a polymer of fucose, xylose, arabinose, galacturonic acid and rhamnose.

Alternatively, the hydrocolloid adhesive agent may be a polymer of acrylic acid or an alkylacrylic acid as defined above. High molecular weight polyacrylic acid is also known as carbomer. Polymers of acrylic acid which may be either crosslinked or uncrosslinked.

Examples of crosslinking agents include allyl ether pentaerythritol, allyl ethers of sucrose or ally ethers of propylene. Crosslinked polymers of acrylic acid are sold under the trade name Carbopol® by Lubrizol Corporation.

The polyacrylic acid may be provided as a salt, such as an ammonium, sodium, potassium, magnesium or calcium salt. Cross-linked polyacrylic acid, such as those cross-linked with divinyl glycol may be provided as salts such as magnesium or calcium salts, particularly calcium salts, also known as polycarbophils. Suitable polycarbophils are sold under the trade name Noveon™ by Lubrizol Corporation.

The gelling agent may be present in a range of from about 0.1 to 10% by dry weight of the tooth whitening film.

The flavouring may be an artificial flavouring or a natural flavouring. The flavouring may be selected from the group comprising menthol, peppermint and an alkyl alkanoate, in which the alkyl group may be straight chained or branched and may comprise from 1 to 8 carbon atoms, and the alkanoate group may comprise from 1 to 5 carbon atoms. Preferably the alkyl group of the alkyl alkanoate has 1, 2, 5 or 8 carbon atoms and the alkanoate group of the alkyl alkanoate may comprise from 1 to 5 carbon atoms, preferably 1, 4 or 5 carbon atoms. Preferred alkyl alkanoates are methyl butyrate [apple/pineapple], ethyl butyrate [orange/pineapple], iosamyl acetate [banana/pear], pentyl butyrate [pear/apricot], pentyl pentanoate [apple], octyl acetate [orange].

Peppermint is a common flavouring for tooth whitening films. Peppermint flavouring contains menthol as well as other components such as menthone, menthofuran, cineol, pulegone, together with some lesser ingredients.

Preferably the flavouring agent is present in a proportion of from about 0.1 to 10 wt.% by dry weight of the tooth whitening film, more preferably from about 1 to 5 wt.% by dry weight of the tooth whitening film.

The sweetener may be a sugar substitute, such as an artificial sugar substitute or a natural sugar substitute. An artificial sugar substitute may be one or more selected from the group comprising sucralose and cyclamate aspartame, advantame, saccharin, acesulfame potassium. A natural sugar substitute may be selected from the group comprising stevia, erythritol, mannitol, sorbital and xylitol. Preferably the sweetener is sucralose.

Preferably the sweetener is present in a proportion of from about 0.01 to 5 wt.% by dry weight of the tooth whitening film. More preferably the acidifier is present in a proportion of from 0.05 to 0.5 wt.% by dry weight of the tooth whitening film.

The acidifier may be an inorganic acidifier or an organic acidifier. Phosphoric acid is a preferred inorganic acidifier. Preferred organic acidifiers are those selected from the group comprising lactic acid, malic acid, acetic acid, and citric acid, with citric acid being most preferred.

Preferably the acidifier is present in a proportion of from about 0.1 to 5 wt.% by dry weight of the tooth whitening film. More preferably the acidifier is present in a proportion of from about 1.0 to 1.5 wt.% by dry weight of the tooth whitening film.

The antioxidant may be one or more selected from tocopherol (vitamin E), tertiary-butylhydroquinone (TBHQ), butylated hydroxyanisole (BHA), butylated hydroxytoluene and ethylenediaminetetraacetic acid or a salt thereof. A preferred antioxidant is ethylenediaminetetraacetic acid (EDTA).

Preferably the antioxidant is present in a proportion of from about 0.01 to 0.5 wt.% by weight of the tooth whitening film, more preferably from about 0.05 to 0.15 wt.% by dry weight of the tooth whitening film.

The chelating agent may be ethylenediaminetetraacetic acid or a salt thereof. Chelating agents can be used to sequester heavy metal ions, thereby preventing the degradation of peroxy carboxylic acids.

The chelating agent may be present in an amount of from about 0.1 to 5% by weight of the dry tooth whitening film.

A preservative may also be present in the tooth whitening film. Typical preservatives may be one or more compounds selected from the group comprising potassium sorbate, benzoic acid and its salts, propionic acid and its salts, salicylic acid and its salts and triclosan.

Preferably the preservative is present in a proportion of from about 0.01 to 5 wt.% by dry weight of the tooth whitening film. More preferably the preservative is present in a proportion of from about 0.05 to 0.5 wt.% by dry weight of the tooth whitening film.

In addition, the dissolvable strip or film may comprise any one of a combination of the following: calcium sodium phosphosilicate such as Vitryxx™; potassium oxalate; the amino acid arginine and related compounds such as arginine bicarbonate; potassium nitrate; and various blends with optional inclusion of a source of fluoride or alternative sources of calcium, sodium, potassium and / or phosphate ions such as calcium phosphate, dicalcium phosphate, tricalcium phosphate, calcium carbonate, calcium silicate, calcium citrate, calcium monofluorophosphate, calcium sulphate, pentasodium triphosphate, sodium hexametaphosphate, sodium metaphosphate, sodium monofluorophosphate, sodium phosphate, sodium polyphosphate, sodium trimetaphosphate, disodium pyrophosphate, tetrasodium pyrophosphate, trisodium phosphate, potassium phosphate, dipotassium phosphate, pentapotassium triphosphate, potassium carbonate, potassium citrate, potassium fluoride, potassium silicate, potassium fluorosilicate, potassium metaphosphate, potassium monofluorophosphate, tetrapotassium pyrophosphate, tripotassium phosphate.

Also disclosed herein is a process for the manufacture of a tooth film. The film does not require the presence of further layers, such as barrier layers or supporting substrates.

The tooth whitening film is prepared from an aqueous tooth whitening liquid. The aqueous tooth whitening liquid is a mixture of the components of the tooth whitening film with water.

The non-hydrogen peroxide bleaching agent and water soluble film-forming polymer components of the tooth whitening film can be mixed with water to provide an aqueous tooth whitening liquid. The water is typically potable water. Distilled or de-ionised water may also be used. Preferably, the components are added to water under shear.

The mixing step may further comprise the addition of one or more further components of the aqueous tooth whitening film. The one or more further components may be selected from one or more of the group comprising colourant, gelling agent, flavouring, sweetener, acidifier, antioxidant and chelating agent.

The mixing parameters, such as shear force and duration, will be dependent upon the scale of the process of manufacture, as well as the component of the tooth whitening film to be mixed. This in turn will affect the choice of equipment to be employed in the mixing step.

Typically, the mixing step is initiated by adding any water soluble film-forming polymer and plasticizer into the water together with any optional gelling agent, further polymers or gums. Typically, any acidifier and any optional further components such as colourant, sweetener or other low level additives also will be added at this stage. The water soluble film-forming polymer and any other polymeric components or gums will be added in an order which prevents an early excessive viscosity build, i.e. the polymers or gums which produce the lowest viscosity build will be added first. Typically, any flavours will be mixed at the latter stages to prevent any degradation of thermally sensitive components or the loss of volatile components. At the laboratory scale, the non-hydrogen peroxide bleaching agent, such as a peroxy carboxylic acid, particularly such as a complex of PAP and polysaccharide such as dextrin, can be incorporated near the end of mixing to prevent unnecessary exposure to heat which may result in degradation. At the industrial scale, the build-up of heat during mixing is significantly reduced, and so the non-hydrogen peroxide bleaching agent, can be added at any point in the mixing step.

For example, the components, can be mixed at a shear rate in the range of from 1,000 to 6,000 rpm. For smaller quantities on a laboratory scale, the shear rate may be in a range of from 2,000 to 6,000 rpm, preferably in a range of from 3,000 to 4,000 rpm. For larger quantities on an industrial scale, the shear rate may be in a range of from 1,500 to 2,500 rpm, preferably approximately 2,000 rpm. The mixing time for smaller quantities on a laboratory scale can be in a range of from about 5 to 20minutes, preferably from about 5 to 10 minutes. For larger quantities on an industrial scale, the mixing time may be in a range of from about 10 to 20 minutes, preferably approximately 15 minutes.

Once the components are mixed with water to provide the aqueous tooth whitening liquid, it may be applied to a substrate. The aqueous tooth whitening liquid may be applied by known film-forming processes, such as dipping, spraying, knife over roller casting, extrusion and injection moulding.

A casting device may be used to apply the aqueous tooth whitening liquid to the substrate such as a caster, spreader, die or hopper. The aqueous liquid may be pumped into a spreader. If necessary, the temperature of the aqueous liquid may be held constant by heating elements in order to carefully control the viscosity of the liquid.

The spreader distributes the aqueous tooth whitening liquid homogeneously over a substrate and controls the thickness profile of the film. The spreader typically has a slot through which the aqueous tooth whitening liquid is cast. The slot is preferably designed to ensure that the hydrostatic pressure between the centre and the edges of the cast film is equilibrated. Preferably, the aqueous tooth whitening liquid is maintained at a constant temperature to ensure a constant viscosity and therefore homogenous thickness distribution of the resulting film.

Many different materials can be used for the substrate such as copper; silver plated copper; chromium plated steel; stainless steel; metal coated with polyvinyl alcohol or gelatin; paper such as silicone coated paper; or polymer films such as polyethylene, polypropylene, polyester such as polyethylene terephthalate (PET), particularly siliconized PET, polytetrafluoroethylene (PTFE) films. Preferably the substrate comprises paper, paper coated with a release layer such as silicone, polyethylene, polypropylene, polytetrafluoroethylene (PTFE) or a polyester such as polyethylene terephthalate (PET), particularly siliconized PET films.

The aqueous tooth whitening liquid carried on the substrate can then be dried. Exemplary drying methods include indirect heating, heating by radiation and air stream drying.

In one embodiment, an air feed stream with no or a low moisture content is directed towards the aqueous tooth whitening liquid on the substrate and an air exhaust stream loaded with water vapour is removed. The air exhaust stream loaded with moisture may be vented or passed to condensers for the condensation and separation of liquid water.

It is possible to dry both surfaces of the aqueous tooth whitening liquid on the substrate. This can be achieved by looping the drying tooth whitening film around a series of polished rollers and directing air feed streams to each exposed surface in turn.

The air feed stream may be at ambient temperature. Alternatively, the air feed stream may be a heated air feed stream. The bleaching agent PAP melts above 75°C and the chemical degrades with the evolution of carbon dioxide to ε-phthalimido pentanol, such that the heated air stream should have a temperature below 60°C. Preferably, the heated air feed stream may have a temperature in the range of from greater than ambient to 60 °C. More preferably, the heated air stream may have a temperature in the range of from 25 °C to 55°C, still more preferably in the range of from 45 to 55 °C. The heating may be carried out in a belt heater with a belt speed in the range of from 0.2 to 2.0 m/min, and preferably at a speed in the range of from 0.5 to 1.5 m/min.

Alternatively, heated rollers or radiative heaters such as infra-red lamps may be used to dry the aqueous liquid to provide the film. These may heat the aqueous tooth whitening liquid carried on the substrate to a temperature in the range of from greater than ambient to about 60°C, preferably to a temperature in the range of from about 25 °C to 55 °C.

The drying step may comprise first drying of a first surface of the aqueous tooth whitening liquid carried on the substrate to provide a first dried tooth whitening film carried on the substrate. The first surface of the aqueous tooth whitening liquid may be the outer surface of the liquid opposite to a second surface adjacent to the support. The first dried tooth whitening film may then be separated from the substrate. The second surface of the first dried tooth whitening film may then be second dried to provide a second dried tooth whitening film. The first and second drying may be independently carried out as discussed above.

In this way, a tooth whitening film can be provided which comprises less than about 15% water by dry weight of the film. The water content is preferably in a range of from about 3 to 12% by dry weight of the film, more preferably in a range of from about 5 to 10% by dry weight of the film and even more preferably in a range of from about 5 to 7% by dry weight of the film.

The tooth whitening film may be stored in sterile packaging, such as a sealed packet. The sealed packet may be water tight, and more preferably water and air tight. The packet may be made of a metallic foil such as aluminium foil, or a plastic film coated with a metallic layer.

The tooth whitening film obtainable by the method disclosed herein may be used in a method of bleaching teeth. The method may comprise at least the step of:

- applying the tooth whitening film, obtained as described herein, to one or more teeth of a subject.

The method may be a cosmetic method. Preferably the method is a solely cosmetic method. As used herein, the term a "solely cosmetic method" means a cosmetic method which does not encompass the treatment or prevention of a medical condition or indication.

Certain aspects and embodiments of the invention will now be illustrated by way of example and with reference to the following tables.

## EXAMPLES

## Reference Example 1

### Hydroxy Apatite (HA) discs: pre-treatment

HA discs are treated prior to use in whitening trials to make them suitable for use.

The treatment involves the formation of a pellicle layer on the surface of the HA substrate followed by exposure to staining broth.

### Pellicle layer formation

Artificial saliva was made using a modified version of WI-82-03-5 Rev 3, where the amount of each reagent was halved to give a half-sized (500 ml) batch of artificial saliva. Porcine gastric mucin (PGM) (1.25 g) was added in 4 parts, mixing with a magnetic stirrer bar at 950 rpm for approximately 15 minutes after each addition and for 90 minutes after the final addition, to yield a cloudy, slightly yellow solution of PGM/artificial saliva mixture (2.5 g/L concentration). HA discs were polished on one side using 240 grit silicon carbide paper and the colour of the polished side measured using a colourimeter according to WI-73-03-7 Rev 2. Each disc was placed polished side up in a petri dish and either PGM/artificial saliva mixture was added to the dish. The dishes were covered with a lid and placed in an oven overnight at about 37°C for about 18 hours. The discs were then removed and patted dry with paper towel, and the colour measured again before use.

### Staining broth recipe and preparation

Preparation of staining broth: Nescafe Gold Blend (registered trade mark) coffee (1 g) was dissolved in boiling water (100 mL) in a 150 mL beaker and stirred with magnetic stirrer bar at 350 rpm for 5 mins.

A single Tetley tea bag was added to boiling water (100 mL) in a 150 mL beaker and stirred with magnetic stirrer bar at 350 rpm for 5 mins. The tea and coffee solutions were added together in a 500 mL beaker.

Concentrated blueberry juice (30 mL) and potassium sorbate (0.5 g) were then added to tea/coffee solution. PGM (0.75 g) was then added to staining broth in three parts after each addition the staining broth was agitated with a magnetic stirrer bar at 950 rpm for 15 minutes, and for 90 minutes after the final addition.

HA discs were each placed in their own petri dish polished side up. The staining broth was added to the dishes until each disc was fully submerged, taking care to avoid pouring the broth straight onto the surface of the discs. The petri dishes were covered with lids, labelled, and put into the oven at about 37°C for approximately 72 hours.

**Teeth whitening strip efficacy**

Stained HA discs are treated using the following protocol:

1. One drop (using a disposable pipette) of room temp. PGM/artificial saliva mix on top of each disc.
2. Square of pre-cut teeth whitening strip placed on top of disc, covering surface.
3. One drop of PGM/artificial saliva mix on top of the strip, spreading out with a paintbrush.
4. After 5 mins, another drop of PGM/artificial saliva mix was added, and surface of discs dabbed with a paintbrush.
5. Step 4 repeated after 10, 15, 20 mins.
6. Discs dabbed with paintbrush after 25 mins (no drop of PGM/artificial saliva at this stage).
7. After 30 mins, discs wiped with paper towel to remove excess whitening strip.
8. Each disc washed in 100 ml water then blotted dry with paper towel.
9. Allow discs to air dry for 1 hour before measuring the colour.

**Colour measurements**

Colour was measured with a colourimeter (Minolta chroma meter CR-400) using the Hunter L, a, b colour scale, where the L value indicates the degree of lightness, with 0 being completely dark and 100 being completely light.
The above procedure was repeated to provide a total of 10 treatments.

**Batch preparation**

Development batches of about 6% $H_2O_2$ teeth whitening strips prepared containing about 6.5% nano-hydroxyapatite paste (n-HAP) and about 6.5% potassium oxalate as outlined by DEV04861 and DEV04852 (see appendix 1). The nano-hydroxyapatite paste (n-HAP) may be NanoXIM (trade mark) These development batches were tested against a standard 6% peroxide formulation to provide a comparative positive control. Two discs were also treated with no whitening strips to act as a negative control.

**Disc treatment**

Two discs were treated by each formulation listed above Table 1 shows which discs were treated by which formulation. Each formulation was assayed by titration with $KMnO_4$.

**Table 1:** Summary of batches tested during whitening trial

| Batch Number | Active ingredient | $H_2O_2$ assay result | Discs treated |
|---|---|---|---|
| DEV04861 | 6% $H_2O_2$ + 6.5% n-HAP paste | 4.95% | A+B |
| DEV04862 | 6% $H_2O_2$ + 6.5% potassium oxalate | 4.91%* | C+D |
| Positive control (19080) | 6% $H_2O_2$ | 5.03% | E + F |
| Negative control | N/A | N/A | G + H |
| *Note: potassium oxalate is also titrated by $KMnO_4$ the value reported is the value corrected for 6.5% oxalate and therefore should be the accurate level of $H_2O_2$. | | | |

The change in lightness (L value) over the course of 10 treatments for each batch tested is shown.
As can be seen, DEV04682 shows the fastest initial rate of whitening. However, after 10 treatments all three peroxide batches reached a similar end points (within ±10% of each other.)
The average L value of the discs treated with DEV04861 and DEV04862 was lower than the starting values for both control samples. A lower starting point may account for a high initial rate of whitening observed for both development batches as the whitening effect may be more profound on darker samples. After three treatments the whitening rates of both development samples are in line with the positive control therefore the addition of n-HAP and potassium oxalate had no impact on the whitening efficacy of the films tested.
The incorporation of tooth desensitising ingredients into a hydrogen peroxide teeth whitening film has had no impact on the whitening efficacy when compared to a standard hydrogen peroxide film.
Both development batches performed significantly better than the negative control. Of the two development batches tested DEV04862 (potassium oxalate) showed an increased whitening rate over the first 4 treatments compared to the control batch. However, after 10 treatments the level of performance was comparable to the control batch.

DEV04861 (n-HAP) performed much more comparatively to the control batch throughout the entire study.

**Reference Example 2**

The applicant has recently carried out development work on a new thin dissolvable film which can reduce/prevent dental hypersensitivity (DH). DH is a common side effect of peroxide teeth whitening However, by incorporating a desensitising ingredient into a thin film alongside a whitening agent it is possible to prevent this undesirable effect.

The applicant has examined the incorporation of three potential desensitising ingredients into thin films with and without whitening agent's present. The three desensitising agents examined are: nano-hydroxyapatite (n-HAP) paste; Vitryxx™ bioactive glass and potassium oxalate.

**Hydroxyapatite**

n-HAP or HAP powder can be incorporated into oral care products such as toothpastes and mouth washes to treat dental sensitivity or to promote remineralisation. Development work was carried out using the applicant's standard 6% hydrogen peroxide teeth-whitening formulation as a starting point however the whitening agent, peroxydone, a polyvinylpyrrolidone-hydrogen peroxide complex was replaced with pure polyvinylpyrrolidone (PVP). Xylitol was employed in the initial formulations in place of sucralose as xylitol promotes mineralization by increasing saliva flow and also has anti-microbial properties. Information on the batches produced is listed below:

DEV04835 and DEV04837 were both attempts to incorporate about 6.5% n-HAP paste (e.g. nanoXIM paste, supplied by Fluidinova) however upon the addition of n-HAP paste the viscosity of casting liquid increased significantly, to the point that the casting liquid was not able to be further processed.

DEV04838 reduced the level of pectin to compensate for the n-HAP however the casting liquid was still too thick to process.

DEV04842 replaced pectin with pullulan however the casting liquid was not thickened by the addition of the n-HAP paste thus implying that the combination of pectin and n-HAP specifically caused the thickening of the casting liquid. As no thickening occurred the casting liquid was too thin to use.

DEV04844 dosed about 10% pectin into the formulation used for DEV04842 thus thickening the formulation enough to allow a film to be produced. Films were of a good quality and contained about 6.5% n-HAP paste.

As DEV04844 successfully produced a high-quality film this formulation was used as the base in an attempt to incorporate hydrogen peroxide.

DEV04848 replaced PVP with peroxydone at the same level as the standard 6% peroxide formulation. The strips produced from DEV04848 were assayed at 4.6% peroxide which is within the limit specified in the applicant's 6% peroxide finished product specification.

DEV04849 was based on about a 10% peroxide formulation and the n-HAP paste was increased to about 10% to provide a high strength high protection strip. No pullulan was present in this film due to the high level of peroxydone required to attain about 10% peroxide in the finished film.

DEV04855 was an attempt to incorporate about 6.5% n-HAP paste into the applicant's non peroxide teeth whitening formulation. Formulation did not thicken upon the addition of the n-HAP paste which again, implies the thickening properties of n-HAP are more impactful when used in conjunction with a pectin-based film. Samples were successfully produced and retained.

**Vitryxx™ Bioactive Glass and Potassium Oxalate**

Alongside n-HAP two other potential ingredients for reducing the sensitising effects of peroxide were examined: Vitryxx™ Bioactive glass and potassium oxalate. Both potassium oxalates and bioactive glasses (e.g. Calcium Sodium Phosphosilicates (CSPS)) have been shown to reduce DH.

DEV04851 and DEV04852 incorporated about 6.5% of Vitryxx™ bioactive glass and potassium oxalate, respectively, in place of n-HAP in a formulation based on DEV04848. Both formulations successfully produced a high-quality film. However, as neither of the actives caused any thickening of the casting liquid, further development may be required. Possible next steps include increasing the pectin level to closer to DEV04835/DEV04837 or increasing the solids content of the casting liquid.

As neither ingredient prevented a film being formed in the formulations attempted both are deemed compatible with the 6% hydrogen peroxide formulation and should therefore also be viable options for 10% peroxide as well.

**Content Range of the Formulations**

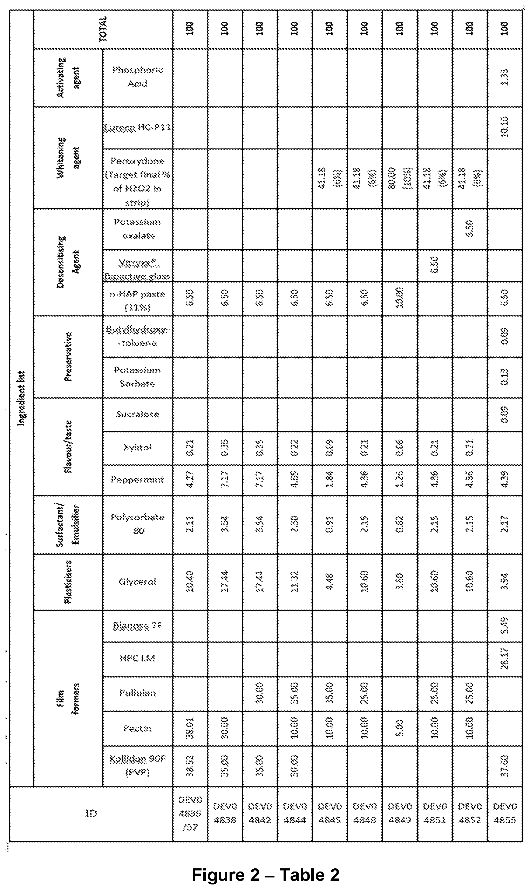

Content range for each of the development tooth sensitivity formulations are presented in Table 2. This is shown in Figure 2.

Table 2 shows the dry weight (wt. %) of ingredients used in the preparation of tooth sensitivity films.

The applicant has developed a thin dissolvable film that contains both a whitening agent and a desensitising agent which has the potential to limit any tooth sensitivity which may be caused by a whitening regime.

Three possible desensitising agents have been successfully employed to date: nano-hydroxyapatite, Vitryxx™ bioactive glass and potassium oxalate. These have been combined with about 6 - 10 wt. % (e.g. 6 wt.% and 10 wt.%) hydrogen peroxide as well as the applicant's non-peroxide (peracid) teeth whitening system.

Of the formulations tested to date DEV04848, DEV04849, DEV04851, DEV04852 and DEV04855 have been the most successful and serve as a strong proof of concept for the incorporation of desensitising agents into teeth whitening thin films.

### Reference Example 3

The below relates to further experiments relating to the preparation of teeth whitening (PAP and $H_2O_2$) and teeth sensitivity formulations that use HAP that is not in nano-form.

The examples in Reference Examples 1 and 2 above relate to using a hydroxyapatite material (n-HAP) that had dimensions on the nano-scale. The n-HAP used was provided in an aqueous dispersion.

In Example 3, there is a discussion of DEV04942 - DEV04944 formulations which are not in nano-form.

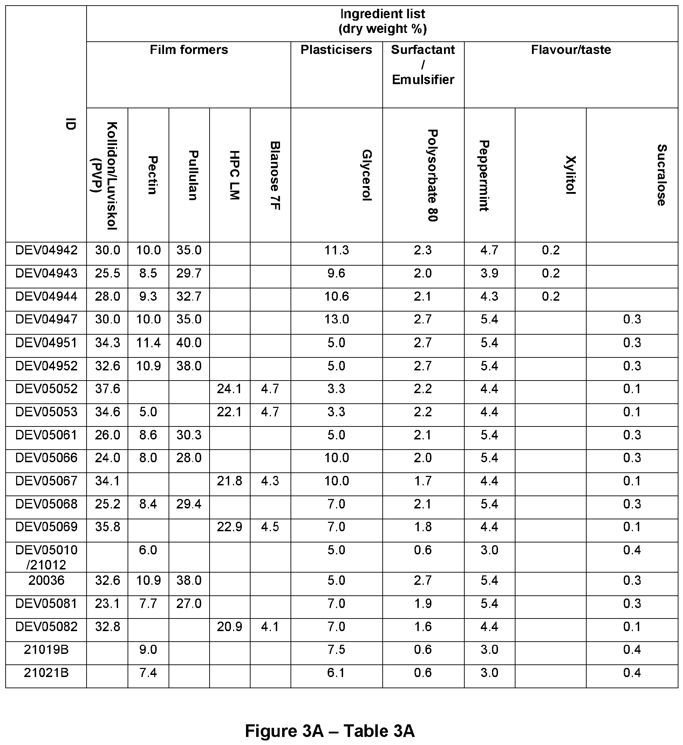

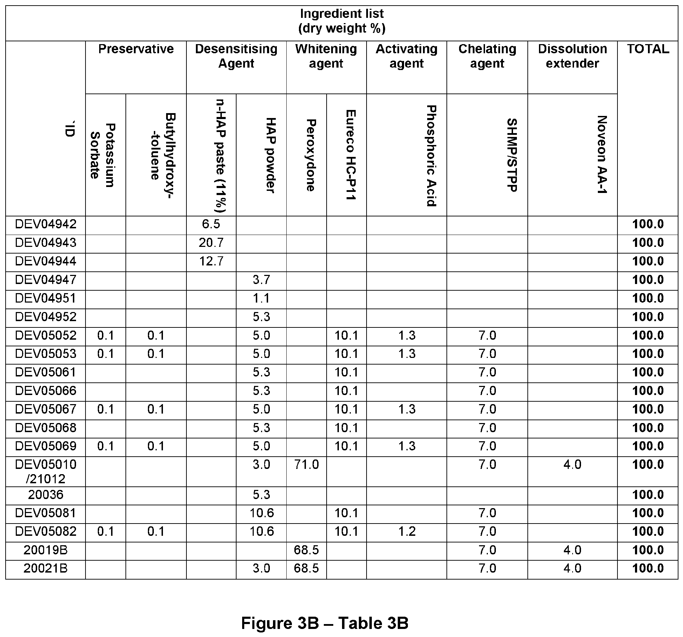

In Figures 3A and 3B, Tables 3A and 3B should be joined together to show all of the ingredients in each of the development products.

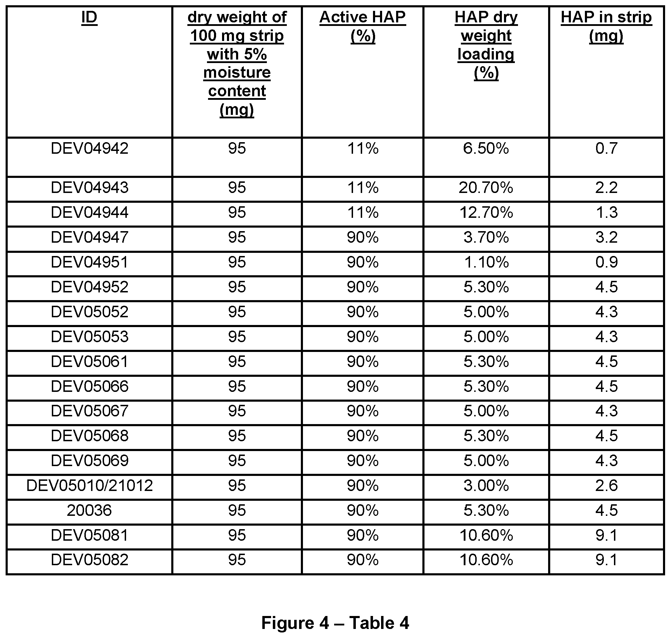

Furthermore, in Figure 4 there is a representation of data for the compositions are shown in Tables 3A and 3B.

### DEV04942-DEV04944

DEV04942 - DEV04944 formulations were used to evaluate the level of n-HAP that could be incorporated into a formulation that combined three main film formers (e.g. PVP, Pectin and Pullulan).

The maximum loading of n-HAP included in the formulation was about 20.7 wt.% and the lowest was about 6.5 wt.%. The exercise demonstrated that there is a limit to the dose of hydroxyapatite that may be achieved if a dilute (e.g. 11 wt.% hydroxyapatite) dispersion is used as the source of hydroxyapatite (3).

**Table 5: theoretical n-HAP dose in development formulations.**

| Formulation ID | Dry weight of n-HAP (wt.%) | n-HAP dose mg/strip[1] |
|---|---|---|
| DEV04942 | 6.5 | 0.7 |
| DEV04943 | 20.7 | 2.2 |
| DEV04944 | 12.7 | 1.3 |
| 1. Dose of n-HAP in each strip based on a theoretical strip weighing 100mg with a water content of 5 wt.%. n-HAP dispersion has a concentration of approximately 11 wt.% n-HAP. | | |

A dry weight of approximately 21 wt.% only affords a theoretical dose of approximately 2 mg of n-HAP in each strip. The requirement for relatively large loadings of n-HAP paste to be used in formulations coupled with unclear regulatory status for nanoparticle containing cosmetics caused the applicant to revaluate the use of n-HAP dispersion paste and focus on HA powder.

### DEV04947, DEV04951 and DEV04952

These formulations were broadly analogous to DEV04942-DEV04944 but the n-HAP was exchanged for HAP powder (e.g. about 90 wt.% active HAP). Three various levels of HAP were included in the formulations and the dose of HAP in each formulation is disclosed in Table 6.

**Table 6: theoretical HAP dose in development formulations.**

| Formulation ID | Dry weight of HAP (wt.%) | HAP dose mg/strip[1] |
|---|---|---|
| DEV04947 | 3.7 | 3.2 |
| DEV04951 | 1.1 | 0.9 |

(continued)

| Formulation ID | Dry weight of HAP (wt.%) | HAP dose mg/strip[1] |
|---|---|---|
| DEV04952 | 5.3 | 4.5 |

1. Dose of n-HAP in each strip based on a theoretical strip weighing 100mg with a water content of 5%. HAP powder has a concentration of approximately 90 wt.% HAP.

The use of a higher active content of HAP powder allowed for a reduced loading of HAP to be added to the formulation. This allows for an increased loading of HAP to be added to the formulation.

**DEV05052 and DEV05053**

These formulations were intended to investigate the addition of HAP powder to the applicant's non-peroxide teeth whitening strip containing PAP and a combination of polyphosphates .

DEV05052 included about 5% of HAP powder and the resulting dried film was found to be extremely brittle. DEV05053 included an additional about 5% pectin to the DEV05052 formulation but this inclusion of pectin had no effect in dealing with the brittleness of the cast film.

These batches indicated that including a concentrated (90 wt.%) source of HAP to the applicant's PAP with polyphosphates formulation may not be a simple process.

**Development production Batch 20036**

This development scale-up batch was based on the DEV04952 development batch. A single 30 kg batch was prepared and coated. The source of HAP was HAP powder and approximately 30 meters of reel stock was collected and processed into strips.

**DEV05061, DEV05066 and DEV05068**

As the inclusion of HAP powder (e.g. about 5 wt.%) was found to cause brittleness in the PAP with polyphosphates strips (DEV05052/3), it was decided to include the active ingredients of the PAP with polyphosphates strips into the DEV04952/20036 formulation. The levels of HAP powder added to all three formulations was set at about 5.3 wt.%.

The main difference between the formulations was defined by the level of glycerol. About a 5% loading of glycerol was found to afford a coated film (DEV05061) that was not of the required quality (uneven drying observed). The level of glycerol was increased to 7 and 10 wt.% (DEV05068 and DEV05066 respectively) and was found to afford films with satisfactory appearance.

The formulations described here indicate that it is possible to prepare a PAP with polyphosphates teeth whitening strip that also contains a desensitising agent (HAP).

**DEV05067 and DEV05069**

To overcome the brittleness observed in DEV05052/3 the level of glycerol was increased to 7 wt. % (DEV05069) and 10 wt.% (DEV05067).

The increase in glycerol here acted to resolve the brittleness observed in DEV05052/3 and the resulting strips with the increased glycerol were found to be of acceptable quality.

**DEV05010/21012**

HAP powder (e.g. about 3 wt.%) was added to the applicant's hydrogen peroxide formulation for whitening strips. There were no issues encountered during the preparation of the development batch.

The formulation was scaled-up and a single 30 kg batch afforded approximately 100 meters of reel stock for further processing into strips.

These developments demonstrated the ability to include HAP powder to hydrogen peroxide teeth whitening strips.

**DEV05081 and DEV05082**

These formulations were intended to investigate the upper limit of the loading of HAP powder that could be readily incorporated into the PAP with polyphosphates formulations.

Both formulations were able to hold about 10.6 wt.% dry weight of HAP powder and it is feasible that up to 20 wt.% could be held with further alterations to the formulation if desired.

### *In-vitro testing*

Two pieces of external in-vitro testing where commissioned with respect to this work. The first was a whitening study to investigate if the inclusion of Hydroxyapatite had any effect on whitening efficacy. The DEV05010 strips that contained about 3 wt.% HAP powder were found to demonstrate whitening efficacy comparable to market leading hydrogen peroxide teeth whitening products that do not contain HAP.

Indeed, the HAP containing strip was faster acting than market leading competitors after one, three and seven treatments. Competitor products were found to be equally efficacious when treatment number was 10 and 14 (Table 7).

**Table 7: Number of R20 Shades Whiter for the Applicant's HAP containing hydrogen peroxide strips (not according to the claims) and market leading hydrogen peroxide competitors.**

| Variable | Treatment | N | Mean | St.Dev | Min | Median | Max |
|---|---|---|---|---|---|---|---|
| 1st Application | Crest 3D WHITE STRIPS CLASSIC WHITE | 6 | 3.17 | 0.95 | 2.08 | 3.18 | 4.53 |
|  | Crest 3D WHITE STRIPS VIVID WHITE | 6 | 2.97 | 0.69 | 1.79 | 3.09 | 3.74 |
|  | DEV05010 | 6 | 4.33 | 1.18 | 2.42 | 4.60 | 5.44 |
| 3rd Application | Crest 3D WHITE STRIPS CLASSIC WHITE | 6 | 4.34 | 0.59 | 3.61 | 4.50 | 4.96 |
|  | Crest 3D WHITE STRIPS VIVID WHITE | 6 | 4.75 | 1.23 | 2.98 | 4.73 | 6.23 |
|  | DEV05010 | 6 | 5.81 | 1.75 | 2.92 | 6.16 | 7.32 |
| 7th Application | Crest 3D WHITE STRIPS CLASSIC WHITE | 6 | 5.88 | 0.67 | 5.01 | 6.01 | 6.69 |
|  | Crest 3D WHITE STRIPS VIVID WHITE | 6 | 5.76 | 1.41 | 3.71 | 5.57 | 7.57 |
|  | DEV05010 | 6 | 6.09 | 1.48 | 3.75 | 5.96 | 8.07 |
| 10th Application | Crest 3D WHITE STRIPS CLASSIC WHITE | 6 | 7.07 | 0.65 | 6.08 | 7.00 | 7.94 |
|  | Crest 3D WHITE STRIPS VIVID WHITE | 6 | 6.58 | 1.32 | 4.70 | 6.36 | 8.13 |
|  | DEV05010 | 6 | 6.37 | 1.69 | 3.40 | 6.65 | 8.05 |
| 14th Application | Crest 3D WHITE STRIPS CLASSIC WHITE | 6 | 7.00 | 0.68 | 6.12 | 6.89 | 7.99 |
|  | Crest 3D WHITE STRIPS VIVID WHITE | 6 | 6.68 | 1.46 | 4.50 | 6.73 | 8.27 |
|  | DEV05010 | 6 | 6.36 | 1.69 | 3.32 | 6.76 | 8.30 |

The second in vitro study assessed whether a hydraulic conductance test could differentiate between the ability of two whitening strips to occlude dentine tubules, whose formulations differ only by the presence or absence of hydroxyapatite. These results are shown below in Table 8.

### **Treatments**

**Table 8: Whitening strip formulation codes**

| Treatment Group | Whitening Strip Formulation |
|---|---|
| **A** | 21019B<br>8% Teeth Whitening |
| **B** | 21021B<br>8% Hydroxyapatite Whitening |

### **Method**

Dentine discs were cut from unrestored, caries-free, coronal portions of human molars. All cuts were made planar perpendicular to the longitudinal axis of each tooth, using a diamond wire saw (approximatethickness: 550 $\mu$m). Three discs passing the baseline criteria were used for each treatment group (n = 3).

Discs were sonicated for 5 minutes in deionized water; rinsed for 30 seconds with deionized water; etchedfor 2 minutes in 10% (w/w) citric acid; rinsed for 30 seconds with deionized water; and pat-dried using absorbent tissue. Etched discs were stored together in deionized water.

A disc was randomly selected and secured within a microfluidic cell, taking care to eliminate any air bubbles or debris. A digital pressure controller was used to force perfusion solution (Earle's Balanced SaltSolution), via a digital flow sensor, through the disc, under a constant pressure of 100 mbar. Mean flow rate was measured over 5 minutes and recorded using direct data export to Microsoft Excel. A mean fluidflow rate within the range 4 to 15 μL/min was deemed acceptable for a baseline measurement. Each discpassing the baseline flow criteria was randomly assigned to a treatment group and subjected to the following treatment regime.

The flow cell was filled with artificial saliva, the reagents for which are listed in Table 9

**Table 9: Artificial saliva reagents**

| Ingredient | Relative Molecular Mass | Concentration |
|---|---|---|
| Potassium Chloride | 74.54 | 30 mM |
| Sodium Chloride | 58.44 | 13 mM |
| Potassium Dihydrogen Orthophosphate | 136.09 | 10 mM |
| Calcium Chloride Dihydrate | 147.02 | 3 mM |
| Type II Porcine Stomach Mucin (M2378 Sigma) | NA | 0.22 % w/w |
| Sodium Azide as preservative | NA | 0.02 % w/w |

A whitening strip was folded gently, immersed fully in the filled cell and left for 30 minutes. The saliva andremains of the strip were subsequently flushed from the cell with deionized water. A post-application, 5-minute mean flow rate measurement was recorded, as previously, and the ability of the test products toocclude dentine tubules calculated.

Flow rate (μL/min) was measured for each of the 3 discs per treatment, across:

- Baseline

- Post-application of whitening strip

For each of the 3 discs per treatment, the percentage flow rate reduction at each timepoint was calculatedas:

$$\% \text{ Flow Rate Reduction} = \frac{(\text{Baseline} - \text{Post-application Timepoint})}{\text{Baseline}} \times 100$$

**Results**

Mean percentage reduction in dentinal microfluidic flow for each whitening strip formulation is presented in Table 10.

**Table 10: Mean percentage flow rate reduction**

| Time Point | Treatment | Mean Reduction in Microfluidic Flow Rate (%) | Standard Deviation |
|---|---|---|---|
| Post-application | 21019B | 37.58 | 2.44 |
| | 21021B | 45.97 | 5.32 |

Both whitening strip formulations caused a reduction in the flow rate of perfusion solution through the dentine tubules. The data indicate that components of both formulae cause some mechanical occlusion when released from the whitening strips.

The whitening strip 21021B, impregnated with hydroxyapatite, achieved a higher mean percentage reduction in microfluidic flow (46%), compared to the non-impregnated 21019B (38%). The data supports the theory that inclusion of hydroxyapatite within a formulation helps the test product to occlude dentinal tubules.

The present applicant has therefore found it is possible to have

- n-HAP formulations that contain a higher loading of n-HAP (e.g. up to about 20.7 wt.%)

- HAP powder formulations at a range of about 1.1 to 10.6 wt.%
- HAP powder formulations that contain whitening agents (e.g. PAP/$H_2O_2$ + P3)

**Claims**

1. A tooth whitening and/or tooth sensitivity strip or film comprising:

    1-20 wt. % hydroxyapatite (HAP);
    at least one or a combination of polyvinylpyrrolidone (PVP) polymers wherein the amount of the PVP polymers is greater than 35 wt.% of the strip or film;
    at least one or a combination of polycarbophil(s), 1-5 wt. % of the strip or film; and
    phthalimido peroxy caproic acid (PAP) with the concentration of: 0.01 wt.% to 5 wt.% of the strip or film;
    wherein the strip or film of has a thickness ranging from 50μm to 500μm.

2. A tooth whitening and/or tooth sensitivity strip or film according to claim 1, wherein the film or strip is dissolvable or substantially dissolvable in an oral cavity.

3. A tooth whitening and/or tooth sensitivity strip or film according to any preceding claim, wherein the amount of hydroxyapatite (HAP) ranges from any of the following: 1 wt.% to 15 wt.% of the strip or film; 1 wt.% to 10 wt.% of the strip or film; or 5 wt.% to 10 wt.% of the strip or film.

4. A tooth whitening and/or tooth sensitivity strip or film according to any preceding claim, wherein the teeth whitening strip or film comprises hydrogen peroxide ($H_2O_2$) with the concentration of hydrogen peroxide ($H_2O_2$) of: 10 wt.% to 30 wt.% of the strip or film; 15 wt.% to 30 wt.% of the strip or film; 10 wt.% to 20 wt.% of the strip or film; or 10 wt.% to 15 wt.% of the strip or film.

5. A tooth whitening and/or tooth sensitivity strip or film according to any preceding claim, wherein the phthalimido peroxy caproic acid (PAP) has a concentration of: 0.02 wt.% to 2 wt.% of the strip or film; 0.03 wt.% to 1.5 wt.% of the strip or film; or 0.05 wt.% to 1.1 wt.% of the strip or film.

6. A tooth whitening and/or tooth sensitivity strip or film according to any preceding claim, further comprising one or more polyphosphates.

7. A tooth whitening and/or tooth sensitivity strip or film according to any preceding claim, wherein the teeth whitening strips or film may comprise a range of other components including any one of or combination of the following:

    - one or more water soluble film-forming polymers;
    - one or more plasticizers;
    - one or more emulsifiers;
    - a source of fluoride; and/or
    - one of more sources of calcium, sodium, potassium and / or phosphate ions.

8. A tooth whitening and/or tooth sensitivity strip or film according to any preceding claim, wherein the film or strip of has a thickness ranging from: 50 μm to 250 μm; 50 μm to 100 μm; or 100 μm to 200 μm.

9. A tooth whitening and/or tooth sensitivity strip or film according to any preceding claim, wherein a soluble film-forming polymer is one or more of the group comprising: pullulan; pectin; starch; dextrin; chitosan; alginic acid; salts of alginic acid and cellulose derivatives.

10. A tooth whitening and/or tooth sensitivity strip or film according to any preceding claim, wherein the hydroxyapatite (HAP) is in the form of a nano crystalline form,
    a paste, or a powder formulation.

11. A tooth whitening and/or tooth sensitivity strip or film according to any preceding claim, wherein the hydroxyapatite (HAP) is in the form of Pentacalcium hydroxide triphosphate e.g. $Ca_{10}(PO_4)_6(OH)_2$.

12. A tooth whitening and/or tooth sensitivity strip or film according to any preceding claim, wherein the hydroxyapatite

EP 4 167 922 B1

(HAP) is in the form of nano-hydroxyapatite (n-HAP) that has a high loading of n-HAP of any of the following: greater than 10 wt.%; greater than 15 weight percent; or up to 20 wt.%.

13. A tooth whitening and/or tooth sensitivity strip or film according to any preceding claim, wherein the hydroxyapatite (HAP) is in the form of nano-hydroxyapatite (n-HAP) that has a high loading of n-HAP of any of the following: 5 - 20 wt.%; 10 - 20 wt.%; or 15 - 20 wt.%.

14. A tooth whitening and/or tooth sensitivity strip or film according to any preceding claim, wherein the hydroxyapatite (HAP) is in the form of a powder formulation and the formulation comprises HAP in the following amounts: 1- 20 wt.%; 1 - 10 wt.%; 5 - 15 wt.%; 5 - 20 wt.%; 1 - 15 wt.%.

15. A process for the manufacture of a tooth whitening strip or film, the process comprising at least the steps of:

- mixing at least one or a combination of polyvinylpyrrolidone (PVP) polymers wherein the amount of the polyvinylpyrrolidone (PVP) polymers is greater than 35 wt.% of the strip or film;
- 1-20 wt.% hydroxyapatite (HAP);
- at least one or a combination of polycarbophil(s), 1-5 wt. % of the strip or film; and
- phthalimido peroxy caproic acid (PAP) with the concentration of: 0.01 wt.% to 5 wt.% of the strip or film;
- with water to provide an aqueous tooth whitening liquid;
- applying the aqueous tooth whitening liquid to a substrate to provide a substrate carrying the aqueous tooth whitening liquid;
- drying the aqueous tooth whitening liquid to provide a tooth whitening film on the substrate, said film having a thickness from 50 $\mu$m to 500 $\mu$m.

**Patentansprüche**

1. Zahnbleichungs- und/oder Zahnempfindlichkeitsstreifen oder -folie, umfassend:

1-20 Gew.-% Hydroxylapatit (HAP);
zumindest ein oder eine Kombination von Polyvinylpyrrolidon(PVP-)Polymeren, wobei die Menge der PVP-Polymere größer als 35 Gew.-% des Streifens oder der Folie ist;
zumindest ein oder eine Kombination von Polycarbophil(en), 1-5 Gew.-% des Streifens oder der Folie; und
Phthalimidoperoxycapronsäure (PAP) mit der Konzentration von: 0,01 Gew.-% bis 5 Gew.-% des Streifens oder der Folie;
wobei der Streifen oder die Folie eine Dicke aufweist, die von 50$\mu$m bis 500$\mu$m reicht.

2. Zahnbleichungs- und/oder Zahnempfindlichkeitsstreifen oder -folie nach Anspruch 1, wobei die Folie oder der Streifen in einer Mundhöhle auflösbar oder im Wesentlichen auflösbar ist.

3. Zahnbleichungs- und/oder Zahnempfindlichkeitsstreifen oder -folie nach einem vorhergehenden Anspruch, wobei die Menge an Hydroxylapatit (HAP) von einem beliebigen des Folgenden reicht: 1 Gew.-% bis 15 Gew.-% des Streifens oder der Folie; 1 Gew.-% bis 10 Gew.-% des Streifens oder der Folie; oder 5 Gew.-% bis 10 Gew.-% des Streifens oder der Folie.

4. Zahnbleichungs- und/oder Zahnempfindlichkeitsstreifen oder -folie nach einem vorhergehenden Anspruch, wobei der/die Zahnbleichungsstreifen oder -folie Wasserstoffperoxid ($H_2O_2$) mit der Konzentrationen von Wasserstoffperoxid ($H_2O_2$) von Folgendem umfasst: 10 Gew.-% bis 30 Gew.-% des Streifens oder der Folie; 15 Gew.-% bis 30 Gew.-% des Streifens oder der Folie; 10 Gew.-% bis 20 Gew.-% des Streifens oder der Folie; oder 10 Gew.-% bis 15 Gew.-% des Streifens oder der Folie.

5. Zahnbleichungs- und/oder Zahnempfindlichkeitsstreifen oder -folie nach einem vorhergehenden Anspruch, wobei die Phthalimidoperoxycapronsäure (PAP) eine Konzentration von Folgendem aufweist: 0,02 Gew.-% bis 2 Gew.-% des Streifens oder der Folie; 0,03 Gew.-% bis 1.5 Gew.-% des Streifens oder der Folie; oder 0,05 Gew.-% bis 1,1 Gew.-% des Streifens oder der Folie.

6. Zahnbleichungs- und/oder Zahnempfindlichkeitsstreifen oder -folie nach einem vorhergehenden Anspruch, ferner umfassend ein oder mehrere Polyphosphate.

7. Zahnbleichungs- und/oder Zahnempfindlichkeitsstreifen oder -folie nach einem vorhergehenden Anspruch, wobei die Zahnbleichungsstreifen oder -folie eine Reihe von anderen Komponenten umfassen kann, beinhaltend ein beliebiges oder eine Kombination des Folgenden:

   - ein oder mehrere wasserlösliche folienbildende Polymere;
   - einen oder mehrere Weichmacher;
   - einen oder mehrere Emulgatoren;
   - eine Quelle von Fluorid; und/oder
   - eine von mehreren Quellen von Calcium-, Natrium-, Kalium- und/oder Phosphationen.

8. Zahnbleichungs- und/oder Zahnempfindlichkeitsstreifen oder -folie nach einem vorhergehenden Anspruch, wobei die Folie oder der Streifen eine Dicke aufweist, die von Folgendem reicht: 50 $\mu$m bis 250 $\mu$m; 50 $\mu$m bis 100 $\mu$m; oder 100 $\mu$m bis 200 $\mu$m.

9. Zahnbleichungs- und/oder Zahnempfindlichkeitsstreifen oder -folie nach einem vorhergehenden Anspruch, wobei ein lösliches folienbildendes Polymer eines oder mehrere aus der Gruppe ist, umfassend: Pullulan; Pektin; Stärke; Dextrin; Chitosan; Alginsäure; Salze von Alginsäure und Cellulosederivate.

10. Zahnbleichungs- und/oder Zahnempfindlichkeitsstreifen oder -folie nach einem vorhergehenden Anspruch, wobei der Hydroxylapatit (HAP) in der Form einer nanokristallinen Form, einer Paste oder einer Pulverformulierung ist.

11. Zahnbleichungs- und/oder Zahnempfindlichkeitsstreifen oder -folie nach einem vorhergehenden Anspruch, wobei der Hydroxylapatit (HAP) in der Form von Pentacalciumhydroxidtriphosphat ist, z. B. $Ca_{10}(PO_4)_6(OH)_2$.

12. Zahnbleichungs- und/oder Zahnempfindlichkeitsstreifen oder -folie nach einem vorhergehenden Anspruch, wobei der Hydroxylapatit (HAP) in der Form von Nano-Hydroxylapatit (n-HAP) ist, der eine hohe Ladung von n-HAP von einem des Folgenden aufweist: größer als 10 Gew.-%; größer als 15 Gewichtsprozent; oder bis zu 20 Gew.-%.

13. Zahnbleichungs- und/oder Zahnempfindlichkeitsstreifen oder -folie nach einem vorhergehenden Anspruch, wobei der Hydroxylapatit (HAP) in der Form von Nano-Hydroxylapatit (n-HAP) ist, der eine hohe Ladung von n-HAP von einem des Folgenden aufweist: 5-20 Gew.-%; 10-20 Gew.-%; oder 15-20 Gew.-%.

14. Zahnbleichungs- und/oder Zahnempfindlichkeitsstreifen oder -folie nach einem vorhergehenden Anspruch, wobei der Hydroxylapatit (HAP) in der Form einer Pulverformulierung ist und die Formulierung HAP in den folgenden Mengen umfasst: 1-20 Gew.-%; 1-10 Gew.-%; 5-15 Gew.-%; 5-20 Gew.-%; 1-15 Gew.-%.

15. Verfahren zur Herstellung eines/einer Zahnbleichungsstreifens oder -folie, wobei das Verfahren zumindest die folgenden Schritte umfasst:

   - Mischen von zumindest einem oder einer Kombination von Polyvinylpyrrolidon(PVP-)Polymeren, wobei die Menge der Polyvinylpyrrolidon(PVP-)Polymere größer als 35 Gew.-% des Streifens oder der Folie ist;
   - 1-20 Gew.-% Hydroxylapatit (HAP);
   - zumindest ein oder eine Kombination von Polycarbophil(en), 1-5 Gew.-% des Streifens oder der Folie; und
   - Phthalimidoperoxycapronsäure (PAP) mit der Konzentration von: 0,01 Gew.-% bis 5 Gew.-% des Streifens oder der Folie;
   - mit Wasser, um eine wässrige Zahnbleichungsflüssigkeit bereitzustellen;
   - Aufbringen der wässrigen Zahnbleichungsflüssigkeit auf ein Substrat, um ein Substrat bereitzustellen, das die wässrige Zahnbleichungsflüssigkeit trägt;
   - Trocknen der wässrigen Zahnbleichungsflüssigkeit, um eine Zahnbleichungsfolie auf dem Substrat bereitzustellen, wobei die Folie eine Dicke von 50 $\mu$m bis 500 $\mu$m aufweist.

**Revendications**

1. Bande ou film pour blanchiment des dents et/ou sensibilité des dents comprenant :

   1 à 20 % en poids d'hydroxyapatite (HAP) ;
   au moins l'un ou une combinaison de polymères de polyvinylpyrrolidone (PVP), dans lesquels la quantité de polymères de PVP est supérieure à 35 % en poids de la bande ou du film ;
au moins l'un ou une combinaison de polycarbophile(s), 1 à 5 % en poids de la bande ou du film ; et
de l'acide phtalimido peroxy caproïque (PAP) avec une concentration de : 0,01 % en poids à 5 % en poids de la bande ou du film ;
dans lesquels la bande ou le film comportent une épaisseur comprise entre 50 $\mu$m et 500 $\mu$m.

2. Bande ou film pour blanchiment des dents et/ou sensibilité des dents selon la revendication 1, dans lesquels le film ou la bande peuvent être solubles ou sensiblement solubles dans une cavité buccale.

3. Bande ou film pour blanchiment des dents et/ou sensibilité des dents selon une quelconque revendication précédente, dans lesquels la quantité d'hydroxyapatite (HAP) est comprise entre l'une quelconque des valeurs suivantes : 1 % en poids à 15 % en poids de la bande ou du film ; 1 % en poids à 10 % en poids de la bande ou du film ; ou 5 % en poids à 10 % en poids de la bande ou du film.

4. Bande ou film pour blanchiment des dents et/ou sensibilité des dents selon une quelconque revendication précédente, dans lesquels la bande ou le film pour blanchiment des dents comprennent du peroxyde d'hydrogène ($H_2O_2$) avec une concentration en peroxyde d'hydrogène ($H_2O_2$) de : 10 % en poids à 30 % en poids de la bande ou du film ; 15 % en poids à 30 % en poids de la bande ou du film ; 10 % en poids à 20 % en poids de la bande ou du film ; ou 10 % en poids à 15 % en poids de la bande ou du film.

5. Bande ou film pour blanchiment des dents et/ou sensibilité des dents selon une quelconque revendication précédente, dans lesquels l'acide phtalimido peroxy caproïque (PAP) comporte une concentration de : 0,02 % en poids à 2 % en poids de la bande ou du film ; 0,03 % en poids à 1,5 % en poids de la bande ou du film ; ou 0,05 % en poids à 1,1 % en poids de la bande ou du film.

6. Bande ou film pour blanchiment des dents et/ou sensibilité des dents selon une quelconque revendication précédente, comprenant en outre un ou plusieurs polyphosphates.

7. Bande ou film pour blanchiment des dents et/ou sensibilité des dents selon une quelconque revendication précédente, dans lesquels les bandes ou le film pour blanchiment des dents peuvent comprendre une gamme d'autres composants comprenant l'un quelconque ou une combinaison des éléments suivants :

   - un ou plusieurs polymères filmogènes solubles dans l'eau ;
   - un ou plusieurs plastifiants ;
   - un ou plusieurs émulsifiants ;
   - une source de fluorure ; et/ou
   - l'une parmi d'autres sources de calcium, de sodium, de potassium et/ou d'ions phosphates.

8. Bande ou film pour blanchiment des dents et/ou sensibilité des dents selon une quelconque revendication précédente, dans lesquels le film ou la bande comportent une épaisseur comprise entre : 50 $\mu$m à 250 $\mu$m; 50 $\mu$m à 100 $\mu$m; ou 100 $\mu$m à 200 $\mu$m.

9. Bande ou film pour blanchiment des dents et/ou sensibilité des dents selon une quelconque revendication précédente, dans lesquels un polymère filmogène soluble est un ou plusieurs membres du groupe comprenant : le pullulane ; la pectine ; l'amidon ; la dextrine ; le chitosane ; l'acide alginique ; les sels d'acide alginique et les dérivés cellulosiques.

10. Bande ou film pour blanchiment des dents et/ou sensibilité des dents selon une quelconque revendication précédente, dans lesquels l'hydroxyapatite (HAP) se présente sous la forme d'une forme nanocristalline, d'une pâte ou d'une formulation en poudre.

11. Bande ou film pour blanchiment des dents et/ou sensibilité des dents selon une quelconque revendication précédente, dans lesquels l'hydroxyapatite (HAP) se présente sous la forme d'hydroxyde triphosphate de pentacalcium, par exemple $Ca_{10}(PO_4)_6(OH)_2$.

12. Bande ou film pour blanchiment des dents et/ou sensibilité des dents selon une quelconque revendication précédente, dans lesquels l'hydroxyapatite (HAP) se présente sous la forme de nano-hydroxyapatite (n-HAP) qui comporte une charge élevée de n-HAP selon l'une quelconque des valeurs suivantes : supérieure à 10 % en poids ;

supérieure à 15 pour cent en poids ; ou jusqu'à 20 % en poids.

13. Bande ou film pour blanchiment des dents et/ou sensibilité des dents selon une quelconque revendication précédente, dans lesquels l'hydroxyapatite (HAP) se présente sous la forme de nano-hydroxyapatite (n-HAP) qui comporte une charge élevée en n-HAP de l'une quelconque des valeurs suivantes : 5 à 20 % en poids ; 10 à 20 % en poids ; ou 15 à 20 % en poids.

14. Bande ou film pour blanchiment des dents et/ou sensibilité des dents selon une quelconque revendication précédente, dans lesquels l'hydroxyapatite (HAP) se présente sous la forme d'une formulation en poudre et la formulation comprend de l'HAP dans les quantités suivantes : 1 à 20 % en poids; 1 à 10 % en poids; 5 à 15 % en poids; 5 à 20 % en poids ; 1 à 15 % en poids.

15. Processus permettant la fabrication d'une bande ou d'un film pour blanchiment des dents, le processus comprenant au moins les étapes de :

- mélange d'au moins l'un ou une combinaison de polymères de polyvinylpyrrolidone (PVP), dans lequel la quantité de polymères de polyvinylpyrrolidone (PVP) est supérieure à 35 % en poids du film ou de la bande ;
- 1 à 20 % en poids d'hydroxyapatite (HAP) ;
- au moins l'un ou une combinaison de polycarbophile(s), 1 à 5 % en poids de la bande ou du film ; et
- de l'acide phtalimido peroxy caproïque (PAP) avec une concentration de : 0,01 % en poids à 5 % en poids de la bande ou du film ;
- avec de l'eau pour fournir un liquide aqueux pour blanchiment des dents ;
- application du liquide de blanchiment des dents aqueux sur un substrat pour fournir un substrat portant le liquide de blanchiment des dents aqueux ;
- séchage du liquide aqueux pour blanchiment des dents afin de fournir un film pour blanchiment des dents sur le substrat, ledit film comportant une épaisseur de 50 $\mu$m à 500 $\mu$m.

**Figure 1 -** Lightness (L) values vs treatment number for each of the batches tested.

| ID | Kollidon 90F (PVP) | Pectin | Pullulan | HPC LM | Biogass 7F | Glycerol | Polysorbate 80 | Peppermint | Xylitol | Sucralose | Potassium Sorbate | Butylhydroxy-toluene | n-HAP paste (33%) | Xirsux Bioactive glass | Potassium oxalate | Peroxydone (Target final % of H2O2 in strip) | Eureca BC-P11 | Phosphoric Acid | TOTAL |
|---|---|---|---|---|---|---|---|---|---|---|---|---|---|---|---|---|---|---|---|
| | Film formers | | | | | Plasticizers | Surfactant/ Emulsifier | Flavour/taste | | | Preservative | | Desensitising Agent | | | Whitening agent | | Activating agent | |
| DEVO 4835/37 | 38.52 | 38.01 | | | | 10.40 | 2.11 | 4.27 | 0.21 | | | | 6.50 | | | | | | 100 |
| DEVO 4838 | 35.00 | 30.00 | | | | 17.44 | 3.54 | 7.17 | 0.35 | | | | 6.50 | | | | | | 100 |
| DEVO 4842 | 35.00 | | 30.00 | | | 17.44 | 3.54 | 7.17 | 0.35 | | | | 6.50 | | | | | | 100 |
| DEVO 4844 | 38.00 | 10.00 | 35.00 | | | 11.32 | 2.30 | 4.65 | 0.22 | | | | 6.50 | | | | | | 100 |
| DEVO 4845 | | 10.00 | 35.00 | | | 4.48 | 0.91 | 1.84 | 0.09 | | | | 6.50 | | | 41.18 (6%) | | | 100 |
| DEVO 4848 | | 10.00 | 25.00 | | | 10.60 | 2.15 | 4.36 | 0.21 | | | | 6.50 | | | 41.18 (6%) | | | 100 |
| DEVO 4849 | | 5.00 | | | | 3.80 | 0.62 | 1.26 | 0.06 | | | | 10.00 | | | 80.00 (10%) | | | 100 |
| DEVO 4851 | | 10.00 | 25.00 | | | 10.60 | 2.15 | 4.36 | 0.21 | | | | | 6.50 | | 41.18 (6%) | | | 100 |
| DEVO 4852 | | 10.00 | 25.00 | | | 10.60 | 2.15 | 4.36 | 0.21 | | | | | | 6.50 | 41.18 (6%) | | | 100 |
| DEVO 4855 | 37.60 | | | 28.17 | 5.49 | 3.94 | 2.17 | 4.39 | | 0.09 | 0.13 | 0.09 | 6.50 | | | | 10.10 | 1.33 | 100 |

Figure 2 – Table 2

| ID | Ingredient list (dry weight %) | | | | | | | | | |
| | Film formers | | | | | Plasticisers | Surfactant / Emulsifier | Flavour/taste | | |
| | Kollidon/Luviskol (PVP) | Pectin | Pullulan | HPC LM | Blanose 7F | Glycerol | Polysorbate 80 | Peppermint | Xylitol | Sucralose |
|---|---|---|---|---|---|---|---|---|---|---|
| DEV04942 | 30.0 | 10.0 | 35.0 | | | 11.3 | 2.3 | 4.7 | 0.2 | |
| DEV04943 | 25.5 | 8.5 | 29.7 | | | 9.6 | 2.0 | 3.9 | 0.2 | |
| DEV04944 | 28.0 | 9.3 | 32.7 | | | 10.6 | 2.1 | 4.3 | 0.2 | |
| DEV04947 | 30.0 | 10.0 | 35.0 | | | 13.0 | 2.7 | 5.4 | | 0.3 |
| DEV04951 | 34.3 | 11.4 | 40.0 | | | 5.0 | 2.7 | 5.4 | | 0.3 |
| DEV04952 | 32.6 | 10.9 | 38.0 | | | 5.0 | 2.7 | 5.4 | | 0.3 |
| DEV05052 | 37.6 | | | 24.1 | 4.7 | 3.3 | 2.2 | 4.4 | | 0.1 |
| DEV05053 | 34.6 | 5.0 | | 22.1 | 4.7 | 3.3 | 2.2 | 4.4 | | 0.1 |
| DEV05061 | 26.0 | 8.6 | 30.3 | | | 5.0 | 2.1 | 5.4 | | 0.3 |
| DEV05066 | 24.0 | 8.0 | 28.0 | | | 10.0 | 2.0 | 5.4 | | 0.3 |
| DEV05067 | 34.1 | | | 21.8 | 4.3 | 10.0 | 1.7 | 4.4 | | 0.1 |
| DEV05068 | 25.2 | 8.4 | 29.4 | | | 7.0 | 2.1 | 5.4 | | 0.3 |
| DEV05069 | 35.8 | | | 22.9 | 4.5 | 7.0 | 1.8 | 4.4 | | 0.1 |
| DEV05010 /21012 | | 6.0 | | | | 5.0 | 0.6 | 3.0 | | 0.4 |
| 20036 | 32.6 | 10.9 | 38.0 | | | 5.0 | 2.7 | 5.4 | | 0.3 |
| DEV05081 | 23.1 | 7.7 | 27.0 | | | 7.0 | 1.9 | 5.4 | | 0.3 |
| DEV05082 | 32.8 | | | 20.9 | 4.1 | 7.0 | 1.6 | 4.4 | | 0.1 |
| 21019B | | 9.0 | | | | 7.5 | 0.6 | 3.0 | | 0.4 |
| 21021B | | 7.4 | | | | 6.1 | 0.6 | 3.0 | | 0.4 |

## Figure 3A – Table 3A

| ID | Preservative | | Desensitising Agent | | Whitening agent | | Activating agent | Chelating agent | Dissolution extender | TOTAL |
|---|---|---|---|---|---|---|---|---|---|---|
| | Potassium Sorbate | Butylhydroxy-toluene | n-HAP paste (11%) | HAP powder | Peroxydone | Eureco HC-P11 | Phosphoric Acid | SHMP/STPP | Noveon AA-1 | |
| DEV04942 | | | 6.5 | | | | | | | **100.0** |
| DEV04943 | | | 20.7 | | | | | | | **100.0** |
| DEV04944 | | | 12.7 | | | | | | | **100.0** |
| DEV04947 | | | | 3.7 | | | | | | **100.0** |
| DEV04951 | | | | 1.1 | | | | | | **100.0** |
| DEV04952 | | | | 5.3 | | | | | | **100.0** |
| DEV05052 | 0.1 | 0.1 | | 5.0 | | 10.1 | 1.3 | 7.0 | | **100.0** |
| DEV05053 | 0.1 | 0.1 | | 5.0 | | 10.1 | 1.3 | 7.0 | | **100.0** |
| DEV05061 | | | | 5.3 | | 10.1 | | 7.0 | | **100.0** |
| DEV05066 | | | | 5.3 | | 10.1 | | 7.0 | | **100.0** |
| DEV05067 | 0.1 | 0.1 | | 5.0 | | 10.1 | 1.3 | 7.0 | | **100.0** |
| DEV05068 | | | | 5.3 | | 10.1 | | 7.0 | | **100.0** |
| DEV05069 | 0.1 | 0.1 | | 5.0 | | 10.1 | 1.3 | 7.0 | | **100.0** |
| DEV05010 /21012 | | | | 3.0 | 71.0 | | | 7.0 | 4.0 | **100.0** |
| 20036 | | | | 5.3 | | | | | | **100.0** |
| DEV05081 | | | | 10.6 | | 10.1 | | 7.0 | | **100.0** |
| DEV05082 | 0.1 | 0.1 | | 10.6 | | 10.1 | 1.2 | 7.0 | | **100.0** |
| 20019B | | | | | 68.5 | | | 7.0 | 4.0 | **100.0** |
| 20021B | | | | 3.0 | 68.5 | | | 7.0 | 4.0 | **100.0** |

Figure 3B – Table 3B

| ID | dry weight of 100 mg strip with 5% moisture content (mg) | Active HAP (%) | HAP dry weight loading (%) | HAP in strip (mg) |
|---|---|---|---|---|
| DEV04942 | 95 | 11% | 6.50% | 0.7 |
| DEV04943 | 95 | 11% | 20.70% | 2.2 |
| DEV04944 | 95 | 11% | 12.70% | 1.3 |
| DEV04947 | 95 | 90% | 3.70% | 3.2 |
| DEV04951 | 95 | 90% | 1.10% | 0.9 |
| DEV04952 | 95 | 90% | 5.30% | 4.5 |
| DEV05052 | 95 | 90% | 5.00% | 4.3 |
| DEV05053 | 95 | 90% | 5.00% | 4.3 |
| DEV05061 | 95 | 90% | 5.30% | 4.5 |
| DEV05066 | 95 | 90% | 5.30% | 4.5 |
| DEV05067 | 95 | 90% | 5.00% | 4.3 |
| DEV05068 | 95 | 90% | 5.30% | 4.5 |
| DEV05069 | 95 | 90% | 5.00% | 4.3 |
| DEV05010/21012 | 95 | 90% | 3.00% | 2.6 |
| 20036 | 95 | 90% | 5.30% | 4.5 |
| DEV05081 | 95 | 90% | 10.60% | 9.1 |
| DEV05082 | 95 | 90% | 10.60% | 9.1 |

**Figure 4 – Table 4**

**EP 4 167 922 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006009737 A
- WO 2005016298 A
- US 2008171000 A
- US 2018289606 A
- GB 2562800 A